# EUROPÄISCHE PATENTANMELDUNG

(11) **EP 3 248 575 A1**
(43) Veröffentlichungstag der Anmeldung: **29.11.2017**
(21) Anmeldenummer: 17170703.7
(22) Anmeldetag: 11.05.2017
(51) Int. Cl.: A61F 2/50, A61F 2/80

(54) **HALBZEUG FÜR EINE PROTHESE, PROTHESE UND VERFAHREN ZUR HERSTELLUNG EINES HALBZEUGS UND EINER PROTHESE**

(30) Priorität: 25.05.2016 DE 102016209101
(71) Anmelder: Burgmann Packings Group GmbH, 74638 Waldenburg (DE)
(72) Erfinder: KRÄMER, Swetlana, 81371 München (DE); SEIDL, Markus, 89257 Illertissen (DE)
(74) Vertreter: Patentanwälte Ruff, Wilhelm, Beier, Dauster & Partner mbB

(57) **Zusammenfassung**

Ein Halbzeug für eine Prothese, insbesondere für eine Unterschenkelprothese, mit einem längserstreckten biegesteifen Schaft weist einen Schlauch aus Fasermaterial in Form von Fasern auf, welcher auf den Schaft aufgebracht ist, ihn umgibt und in wenigstens einem Mittelbereich des Schaftes mit ihm unlösbar verbunden ist. Der Schlauch weist einen freien, beweglichen und biegeschlaffen Freibereich auf, welcher über mindestens ein erstes Ende des Schaftes übersteht. Der Freibereich kann zur Herstellung einer Prothese mit dem Halbzeug auf einen Aufnahmeköcher für einen Gliedstumpf aufgelegt werden und mittels Harz verbunden bzw. angeklebt werden.

## Beschreibung

### ANWENDUNGSGEBIET UND STAND DER TECHNIK

Die Erfindung betrifft ein Halbzeug für eine Prothese, insbesondere für eine Unterschenkelprothese, mit einem längserstreckten biegesteifen Schaft. Weiterhin betrifft die Erfindung eine Prothese, die mit einem solchen Halbzeug hergestellt ist, ein Verfahren zur Herstellung eines solchen Halbzeugs sowie ein Verfahren zur Herstellung einer solchen Prothese mit diesem Halbzeug.

Ein Halbzeug für eine Prothese mit einem längserstreckten biegesteifen Schaft ist bekannt und weist einen unteren und einen oberen Bereich zur Verbindung mit jeweils einem Adapterelement auf. Mittels der Adapterelemente wird in dem oberen Bereich eine Verbindung zu einem Aufnahmeköcher für einen Gliedstumpf und in dem unteren Bereich eine Verbindung zu einer Fußprothese hergestellt.

Wenig vorteilhaft bei dem bekannten Halbzeug ist, dass die Verbindung zu dem Aufnahmeköcher über ein Adapterelement hergestellt wird, welches meist aus einem hochlegierten Stahl hergestellt ist. Dies führt zu einem hohen Gewicht einer mit dem bekannten Halbzeug hergestellten Prothese. Eine schwere Prothese schränkt die Bewegungsfreiheit von Prothesenträgern ein und mindert deren Lebensqualität. Zudem weisen viele hochlegierte Stähle eine geringe Chlorbeständigkeit auf, so dass ein Einsatz der Prothesen mit Adapterelementen aus hochlegierten Stählen in chlorhaltigen Medien wie bspw. gechlorten Schwimmbecken nicht empfehlenswert ist.

### AUFGABE UND LÖSUNG

Aufgabe der Erfindung ist es, ein vorgenanntes Halbzeug für eine Prothese, eine vorgenannte Prothese sowie Verfahren zur Herstellung von Halbzeug und Prothese bereitzustellen, mit denen Probleme des Standes der Technik gelöst werden können und es insbesondere möglich ist, eine gewichtsoptimierte Prothese bei vergleichbarer Funktionalität und mit verbesserter Medienbeständigkeit herzustellen.

Gelöst wird diese Aufgabe durch ein Halbzeug mit den Merkmalen des Anspruchs 1, eine Prothese mit den Merkmalen des Anspruchs 10, ein Verfahren zur Herstellung eines solchen Halbzeugs mit den Merkmalen des Anspruchs 13 oder 14 sowie ein Verfahren zur Herstellung einer solchen Prothese mit diesem Halbzeug mit den Merkmalen des Anspruchs 15. Vorteilhafte sowie bevorzugte Ausgestaltungen der Erfindung sind Gegenstand der weiteren Ansprüche und werden im Folgenden näher erläutert. Dabei werden manche der Merkmale nur für das Halbzeug, die Prothese oder eines der Verfahren beschrieben. Sie sollen jedoch unabhängig davon sowohl für das Halbzeug und die Prothese als auch für eines der Verfahren selbständig und unabhängig voneinander gelten können. Der Wortlaut der Ansprüche wird durch ausdrückliche Bezugnahme zum Inhalt der Beschreibung gemacht.

Das Halbzeug weist neben dem längserstreckten bzw. länglichen biegesteifen Schaft einen Schlauch aus Fasermaterial auf, vorteilhaft in Form von Faserrovings, welcher an dem Schaft angebracht ist, innen und/oder außen an ihm anliegt und in wenigstens einem Mittelbereich des Schaftes mit ihm unlösbar verbunden ist. Der Schlauch weist auch einen freien, beweglichen und biegeschlaffen Freibereich auf, welcher über mindestens ein erstes Ende des Schaftes übersteht, vorteilhaft ein freies Ende.

Der längserstreckte bzw. längliche oder lange Schaft kann ein Profillängsmaterial mit einem beliebigen Querschnitt sein, bspw. ein Vollmaterial oder vorteilhaft ein Rohr. Bevorzugt wird der Schaft von einem Rohr mit rundem bzw. kreisrundem Querschnitt und einem Durchmesser zwischen 25 mm und 40 mm gebildet.

Der Schaft und/oder der Schlauch können aus Faserfilamenten bestehen, bevorzugt aus Kohlefasern, Aramidfasern, Glasfasern, Basaltfasern, einer Kombination dieser Fasern oder einer Kombination der genannten Fasern mit anderen Fasern. Die Fasern weisen eine hohe chemische Beständigkeit auf, wodurch sie sich auch für einen Einsatz in einem korrosiven Umfeld eignen, bspw. in gechlortem Wasser bei einer Verwendung in Badeprothesen. Zudem weisen die Fasern mitsamt einem geeigneten Klebstoff oder Harz ein geringes Gewicht und eine hohe Festigkeit auf, so dass eine erhebliche Gewichtsersparnis der Prothese erreicht werden kann.

Für eine Herstellung des Schafts und/oder des Schlauchs eignen sich mono-, bi- oder multiaxiale Gelege aus Fasern bzw. Chemiefasern, evtl. auch Faserrovings. Die Verwendung von gewebten, gewickelten, geflochtenen oder miteinander vernähten Fasern bzw. Faserrovings mit schrägen oder unterschiedlich verlaufenden Faserwinkeln ermöglicht es, dass ein solcher Schlauch sehr flexibel gestaltet ist und leicht in seinem Durchmesser verändert bzw. angepasst werden kann, was auch reversibel sein kann. Der Schlauch kann dabei sowohl um eine Längsachse bzgl. des Schafts in sich geschlossen als auch mit einem Einschnitt versehen sein. Der Schlauch kann auch von mehreren längserstreckten Streifen gebildet werden, die sich als der Freibereich in eine Längsrichtung des Schafts erstrecken. Der Schlauch kann bzgl. der Längsachse des Schafts einen ähnlichen Querschnitt wie der Schaft oder einen davon abweichenden Querschnitt aufweisen. Bevorzugt weist er einen etwas größeren Querschnitt als der Schaft auf.

Der Freibereich wird von einem losen biegeschlaffen Schlauchabschnitt gebildet, der einen Übergangsbereich des Schafts überlappt und über diesen übersteht. Ein Mittelbereich wird von dem fest mit dem Schlauch verbundenen Schaft gebildet. Der Freibereich des Schlauchs kann aus wenigstens einer Lage bestehen, mit wenigstens einem Abschnitt des Mittelbereichs unlösbar verbunden sein und wenigstens eine oberste bzw. äußerste und/oder innerste Lage des Mittelbereichs bilden. Alternativ kann der Freibereich wie der Schlauch auch mehrlagig ausgebildet sein.

In Ausgestaltung der Erfindung weist der Schaft an seinem ersten Ende einen Übergangsbereich auf, welcher sich über 5 % bis 20 % einer Schaftlänge erstreckt. In diesem überlappt der Schlauch den Schaft frei, beweglich und biegeschlaff, wobei vorzugsweise der Übergangsbereich direkt an den Mittelbereich anschließt.

Der biegesteife Schaft weist den vorgenannten Übergangsbereich auf, der von dem biegeschlaffen Schlauch lose überlappt wird. Der Übergangsbereich kann den gleichen Querschnitt wie der Mittelbereich des Schafts aufweisen, kann jedoch auch in seinem Querschnitt abweichen. Beispielsweise kann der Übergangsbereich eine höhere oder geringe Wandstärke als der restliche Schaft aufweisen und/oder größere oder kleinere Innen- und/oder Außenabmessungen. Dies ermöglicht vielfache Gestaltungsmöglichkeiten, um eine Funktionalität und ein Design zu beeinflussen.

In weiterer Ausgestaltung liegt der Schlauch in dem Mittelbereich umlaufend eng an einer Innen- und/oder Außenseite an dem Schaft an. Vorteilhaft ist er dabei daran befestigt oder festgeklebt.

Der Schaft weist den Mittelbereich auf, in dem der Schaft mit dem Schlauch umlaufend und unlösbar eng verbunden ist. Eine unlösbare Verbindung des Schafts und des Schlauchs kann bspw. durch ein Verkleben oder Verschweißen herbeigeführt werden.

In weiterer Ausgestaltung erstreckt sich der Schlauch über den Mittelbereich des Schafts hinaus bis zu einem dem Freibereich abgewandten zweiten freien Ende des Schaftes.

Der Mittelbereich des Schafts kann sich biegesteif bis zu dem zweiten Ende des Schafts, welches auf einer dem Freibereich abgewandten Seite des Halbzeugs angeordnet ist, erstrecken. Somit kann er die Anbringung von Verbindungselementen zu bspw. einer Fußprothese ermöglichen.

In weiterer Ausgestaltung steht der Schlauch an dem zweiten Ende des Schafts ebenfalls mit einem zweiten freien, beweglichen und biegeschlaffen Freibereich über den Schaft über, insbesondere mit einer überstehenden Länge von mindestens 10% der Länge des Schafts.

Der zweite Freibereich kann einen eventuell vorhandenen zweiten Übergangsbereich überlappen und zur direkten oder indirekten Verbindung des zweiten Endes des Schafts oder des zweiten Übergangsbereichs an die Fußprothese dienen.

In weiterer Ausgestaltung ist an dem Ende des Schafts mit dem Freibereich eine Aufnahmeschale mit einer Erstreckungsebene im Wesentlichen quer zur Längsrichtung des Schafts angeordnet, welche auf einer dem Schaft abgewandten Seite eine konkave Krümmung aufweist. Ein Durchmesser der Aufnahmeschale kann den Durchmesser des Schaftes übersteigen, vorzugsweise um 20 % bis 100 %.

Bevorzugt ist die Aufnahmeschale aus Kunststoff oder einem faserverstärkten Kunststoff hergestellt. Eine konkav gekrümmte Innenkontur der Aufnahmeschale entspricht dabei vorteilhaft einer konvexen Außenkontur des Aufnahmeköchers. In weiterer Ausgestaltung der Erfindung liegt der Aufnahmeköcher mit einem ersten, unteren Ende an der vorgenannten Aufnahmeschale an und ist vorzugsweise mit dieser verklebt.

Der Aufnahmeköcher kann von der Aufnahmeschale abschnittsweise umschlossen werden. Durch sich ergänzende Konturen des Aufnahmeköchers und der Aufnahmeschale kann der Aufnahmeköcher vorteilhaft in der Aufnahmeschale stabilisiert und ausgerichtet werden.

In weiterer Ausgestaltung ist die Aufnahmeschale im Wesentlichen rotationssymmetrisch und dauerhaft mit dem Schaft verbunden und eine konkave Vertiefung der Aufnahmeschale beträgt zwischen 10% und 30% des Durchmessers des Schafts. Sie kann angeklebt sein, beispielsweise mit 2-K-Kleber, der schnellhärtend sein kann. Es ist auch möglich, die Aufnahmeschale als Bestandteil des Halbzeugs herzustellen bzw. eine gesamte Baueinheit auszuliefern. So kann der Schritt des Verbindens bzw. Verklebens beim Anwender entfallen.

Die Aufnahmeschale weist Abschnitte auf, die im Wesentlichen rotationssymmetrisch zu einer Längsachse des Schafts oder einer Achse parallel zur Längsachse des Schafts sind. Die Aufnahmeschale kann Unterbrechungen oder Ausbrüche aufweisen, um vorteilhafterweise zu einer Gewichtsersparnis beizutragen. Ein der konkaven Vertiefung abgewandter Bereich der Aufnahmeschale kann einen zylindrischen Abschnitt aufweisen, welcher dauerhaft mit dem Schaft verbunden ist, bspw. verklebt oder verschweißt. Eine Verbindung zwischen Schaft und Aufnahmeschale kann auch durch einen Formschluss wie bspw. eine Übermaßpassung oder eine Verschraubung geschaffen werden.

In weiterer Ausgestaltung ist an dem zweiten bzw. unteren freien Ende des Schafts ein Befestigungsteil angeordnet, das insbesondere am Schaft befestigt ist und einen größeren Querschnitt als der Schaft aufweist. Das Befestigungsteil kann durch einen runden Rohrabschnitt gebildet werden, jedoch sind auch andere Querschnitte möglich, bspw. ein rechteckiger Querschnitt. Mit dem Befestigungsteil kann eine Verbindung zu der Fußprothese ermöglicht werden. Vorteilhafterweise weist das Befestigungsteil eine Einstellvorrichtung auf. Somit sind durch das Befestigungsteil Verbindungs- und Einstellmöglichkeiten für die Fußprothese gegeben. Die Einstellvorrichtung kann Justierelemente wie bspw. Justierschrauben umfassen.

In weiterer Ausgestaltung steht der Freibereich mit einer Länge von mindestens 20% der Länge des Schafts bzw. 10 cm bis 30 cm über das Ende des Schaftes über, vorzugsweise mindestens 50%. Ein großer Überstand des Freibereichs über das Ende des Schafts ermöglicht eine große Klebefläche und somit eine sichere und haltbare Verbindung.

In weiterer Ausgestaltung kann der Schlauch wenigstens einlagig sein, insbesondere zwei- oder mehrlagig. Über eine Variation von Schlauchlagen bzw. deren Anzahl kann eine Anpassung an ein Körpergewicht eines Prothesenträgers vorgenommen werden. Bei leichtgewichtigen Prothesenträgern kann eine geringe Anzahl von Schlauchlagen Anwendung finden, bei schwergewichtigen Prothesenträgern eine Vielzahl von Schlauchlagen. Somit lässt sich eine individuell an den Prothesenträger angepasste Prothese schaffen, welche bei leichtgewichtigen Prothesenträgern ein unnötig hohes Gewicht der Prothese vermeidet und bei schwergewichtigen Prothesenträgern ausreichend Festigkeit bietet.

Am oberen Ende des Schafts einer erfindungsgemäßen Prothese ist ein Aufnahmeköcher für einen Gliedstumpf befestigt. Die Faserrovings des Freibereichs erstrecken sich über mindestens 20% der Höhe des Aufnahmeköchers über eine Außenseite des Aufnahmeköchers, wobei sie auf der Außenseite aufliegen und mit dieser verbunden sind.

Die Prothese kann sowohl eine Unterschenkelprothese als auch eine Oberschenkelprothese sein. Als Verbindungsmöglichkeiten des Freibereichs des Schlauchs mit der Außenseite des Aufnahmeköchers kommen stoffschlüssige Verfahren in Frage, beispielsweise ein Verkleben mit einem Harz an der Atmosphäre oder in einer evakuierten Vorrichtung.

In weiterer Ausgestaltung der Erfindung ist an einem zweiten Ende des Schafts ein Fußteil befestigt, wobei die Faserrovings eines vorgenannten unteren Freibereichs das Fußteil überlappen, vorzugsweise auf der Außenseite des Fußteils aufliegen und daran befestigt sind. Das Fußteil kann ein gelenkiges Element aufweisen, um eine Beweglichkeit der Fußprothese zu ermöglichen.

In weiterer Ausgestaltung der Erfindung ist das Fußteil in eine Fußhülle eingesteckt, wobei vorzugsweise die Fußhülle einem menschlichen Fuß nachgebildet ist. Das Fußteil und die Fußhülle bilden die Fußprothese, die funktionelle und optische Eigenschaften bereitstellt. Durch die Fußhülle können Laufeigenschaften eines natürlichen Gehapparates simuliert werden und die Fußhülle kann zudem vorteilhaft zu einer optischen Angleichung an einen natürlichen Fuß beitragen.

Bei einem erfindungsgemäßen Verfahren zum Herstellen eines Halbzeugs werden der längserstreckte biegesteife Schaft und der biegeschlaffe Schlauch aus Fasermaterial in Form der Fasern bzw. Faserrovings bereitgestellt. Der Schlauch wird auf dem Schaft in wenigstens dem Mittelbereich des Schafts befestigt, wobei der Schlauch mit dem freien, beweglichen und biegeschlaffen Freibereich über das erste Ende des Schafts übersteht.

Der separat hergestellte Schlauch aus Fasermaterial kann auf den separat hergestellten Schaft aufgebracht werden, wobei der Übergangsbereich des Schafts ausgespart werden kann. Der Schlauch und der Schaft können vorteilhaft miteinander verklebt werden, bspw. mittels eines Harzes, bevorzugt in einem Vakuumverfahren.

Alternativ kann bei einem erfindungsgemäßen Verfahren zum Herstellen eines Halbzeugs der Schaft in einer entsprechenden Vorrichtung samt dem Mittelbereich, dem Übergangsbereich und dem Freibereich in einem Stück bzw. gleichzeitig, also in einem einzigen Laminierschritt, hergestellt werden. So können Zeit und Aufwand eingespart werden. Dazu erfolgt zuerst ein Bereitstellen eines Schlauchs aus Fasermaterial. Dann erfolgt ein Herstellen eines längserstreckten biegesteifen Schafts aus einem Längsbereich des Schlauchs durch Verkleben bzw. auf übliche Art mittels eines Harzes. Dabei wird der restliche Längsbereich des Schlauchs als freier, beweglicher und biegeschlaffer Freibereich freigelassen, der über das erste Ende des Schafts übersteht.

Bei einem erfindungsgemäßen Verfahren zum Herstellen einer Prothese mittels des Halbzeugs wird der Aufnahmeköcher an dem freien Ende des Schafts in genau vorgegebener Position und Lage befestigt. Dann erfolgt das Anlegen der Fasern des freien, beweglichen und biegeschlaffen Freibereichs an der Außenseite des Aufnahmeköchers, und dann das Verbinden des Freibereichs mit dem Schaft und dem Übergangsbereich sowie mit dem Aufnahmeköcher, evtl. auch mit einer vorgenannten Aufnahmeschale.

Eine Verbindung zwischen der Aufnahmeschale und dem Aufnahmeköcher kann vorteilhaft durch ein Verkleben hergestellt werden, bevorzugt mittels eines schnell härtenden Klebers oder Harzes.

In weiterer Ausgestaltung kann vor oder nach dem Anlegen und Verkleben der Fasern des Schlauchs an der Außenseite des Aufnahmeköchers der Freibereich des Schlauchs beschnitten und an den Aufnahmeköcher angepasst werden.

Diese und weitere Merkmale gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können, für die hier Schutz beansprucht wird. Die Unterteilung der Anmeldung in einzelnen Abschnitte sowie Zwischen-Überschriften beschränken die unter diesen gemachten Aussagen nicht in ihrer Allgemeingültigkeit.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind. Dabei zeigen:
- Fig. 1: ein erfindungsgemäßes Halbzeug mit einem längserstreckten biegesteifen Schaft sowie mit einem biegeschlaffen Freibereich,
- Fig. 2: das Halbzeug von Fig. 1 mit einem freigelegten Übergangsbereich,
- Fig. 3: das Halbzeug von Fig. 2 mit einer Aufnahmeschale,
- Fig. 4: das Halbzeug und die Aufnahmeschale von Fig. 3 samt einem Aufnahmeköcher und
- Fig. 5: eine erfindungsgemäße Unterschenkelprothese mit dem Halbzeug von Fig. 4 sowie mit einem Befestigungsteil und einer Fußprothese.

### DETAILLIERTE BESCHREIBUNG DER AUSFÜHRUNGSBEISPIELE

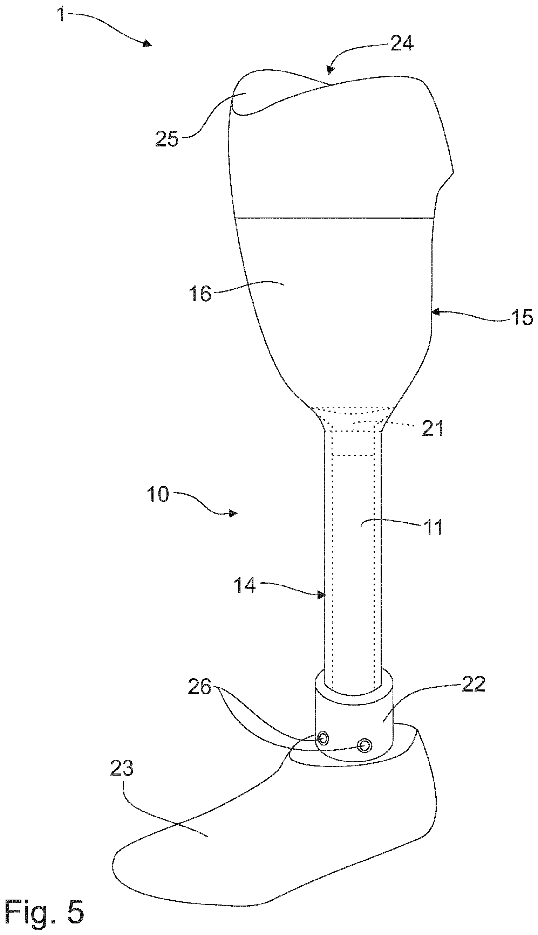

Eine in der Fig. 5 dargestellte Unterschenkelprothese 1 dient einem Ersatz fehlender Gliedmaßen und einer Herstellung bzw. einer Wiederherstellung von funktionellen Eigenschaften eines menschlichen Bewegungsapparates. Die erfindungsgemäße Unterschenkelprothese 1 weist ein verarbeitetes erfindungsgemäßes Halbzeug 10, einen Aufnahmeköcher 25 und eine Fußprothese 23 auf. Der Aufnahmeköcher 25 weist in einem oberen, also dem Halbzeug 10 abgewandten, Bereich einen Einstieg 24 auf, welcher einer Verbindung mit einem Gliedstumpf dient. Am unteren Ende des Halbzeugs 10, der dem Aufnahmeköcher 25 abgewandt ist, ist die Fußprothese 23 angeordnet.

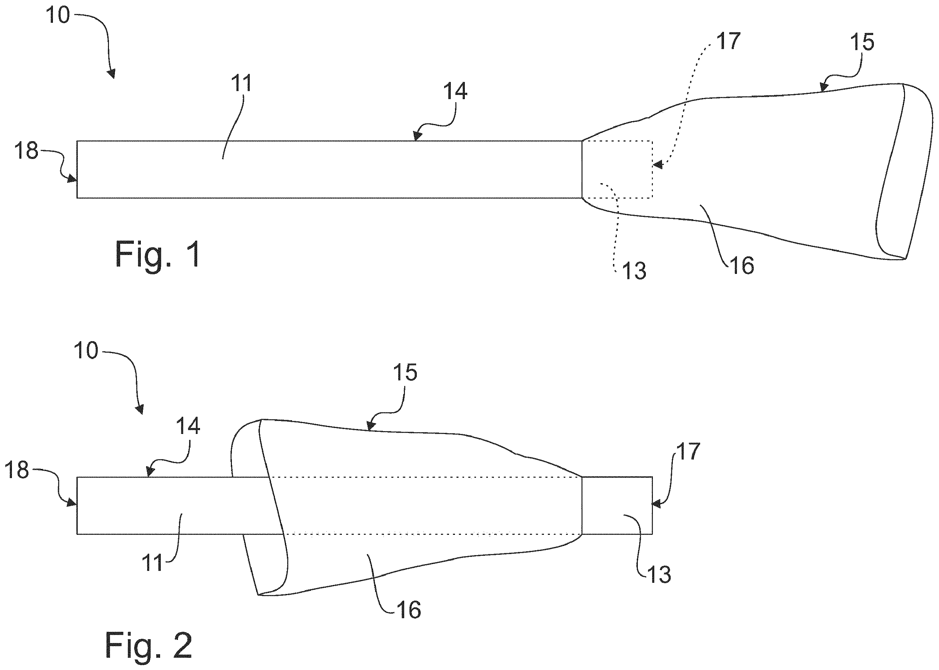

Ein erfindungsgemäßes Halbzeug gemäß Fig. 1 weist einen biegesteifen längserstreckten bzw. länglichen Schaft 14 auf, welcher einen Mittelbereich 11, einen Übergangsbereich 13 und einen losen, biegeschlaffen Schlauch 15 aufweist. Der Schaft 14 ist von einem runden Rohr gebildet, vorteilhaft aus Faserverbundmaterial, welches bevorzugt einen Außendurchmesser von 25 mm bis 40 mm aufweist. Der Schlauch 15 ist als ein röhrenförmiges, flexibles Flächengebilde ausgeführt, welches einen größeren Querschnitt als das Rohr aufweist. Alternativ kann der Schlauch 15 einen kleineren Durchmesser als der Schaft 14 aufweisen oder einen identischen Durchmesser wie der Schaft 14 und aufweitbar sein. Er besteht aus einem vorbeschriebenen Fasermaterial bzw. aus Chemiefaserfilamenten. Er kann auch aus Faserrovings gewebt sein für eine flexible Formgebung und vor allem gute Anpassung des Durchmessers bzw. Durchmesserverlaufs.

In dem Mittelbereich 11 ist der Schlauch 15 fest mit dem Schaft 14 verbunden, insbesondere verklebt. In dem Übergangsbereich 13 ist der Schaft 14 von dem biegeschlaffen losen Schlauch 15, nämlich einem Freibereich 16, überlappt. Der Schlauch weist einen größeren Querschnitt bzgl. einer Längsachse des Schafts 14 auf als der Schaft 14 und erstreckt sich an einem ersten Ende 17 des Schafts 14 über den Übergangsbereichs 13 und über den Schaft 14 hinaus. Der Übergangsbereich 13 ist ein einstückiger Bestandteil des Schafts 14, welcher nicht mit dem Schlauch 15 verbunden ist. Auf der gegenüberliegenden Seite des Schafts 14 schließt der Schlauch 15 mit einem zweiten Ende 18 des Schafts 14, hier dem Mittelbereich 11, ab.

In Fig. 2 ist das Halbzeug 10 mit zurückgeschlagenem Freibereich 16 dargestellt, so dass der Übergangsbereich 13 des Schafts 14 freiliegt. Der Freibereich 16 ist hier über den Mittelbereich 11 des Schafts 14 gestülpt. Der Schlauch 15, bzw. der Freibereich 16 können wenigstens eine Lage Faserrovings aufweisen, bevorzugt weisen sie jedoch zwei oder mehr Lagen Faserrovings auf.

Die Unterschenkelprothese 1 weist gemäß Fig. 5 innenliegend in dem Einstiegsbereich 24 den Aufnahmeköcher 25 auf. Der Aufnahmeköcher 25 ist von dem Schlauch 15 des Halbzeugs 10 umgeben. Der Freibereich 13 dient einer Verbindung des Halbzeugs 10 und des separat hergestellten und an den Gliedstumpf individuell angepassten Aufnahmeköchers 25. Der Freibereich 16 ist über eine Außenseite des Aufnahmeköchers 25 überlappt und mit diesem verklebt. Der Mittelbereich 11 des Schafts 14 dient einer direkten oder indirekten Verbindung des Halbzeugs 10 mit einer Fußprothese 23.

Ein unteres, zweites Ende 18 des Schafts 14 weist ein Befestigungsteil 22 auf. Dieses ist als ein Rohrabschnitt mit Justierelementen 26 dargestellt und umschließt einen unteren Abschnitt des Schafts 14. Die Justierelemente 26 sind um 90 ° versetzt um einen Umfang des Befestigungsteils angeordnet. In Fig. 5 sind zwei Justierelemente 26 sichtbar, vorteilhafter Weise sind an dem Befestigungsteil 22 vier Justierelemente 26 angeordnet.

Zur Herstellung eines erfindungsgemäßen Halbzeugs 10 gemäß den Fig. 1 und 2 wird ein Schlauch 15 mit einem Profillängsmaterial wie dem hier beispielhaft dargestellten runden Rohr als Schaft in einem Abschnitt des Mittelbereichs 11 bis hin zum Übergangsbereich 13 verbunden. Der Schlauch 15 kann dazu um eine Längsachse des Profillängsmaterials vollumfänglich geschlossen sein oder auch Unterbrechungen und Einschnitte aufweisen. Bevorzugt weist der Schlauch Faserrovings aus Glasfasern, Kohlenfasern, Aramidfasern oder Basaltfasern, eine Kombination dieser Fasern oder Anteile dieser Fasern auf. Das Profillängsmaterial ist bevorzugt ein Rundrohr und weist einen Außendurchmesser von 25 mm bis 40 mm auf.

Der Schlauch 15 wird in dem Mittelbereich 11, welcher sich von dem zweiten Ende 18 des Schafts 14 bis zu dem Übergangsbereich 13 erstreckt, mit dem Schaft 14 unlösbar verbunden.

Der Übergangsbereich 13 erstreckt sich an dem ersten Ende 17 des Schafts 14 über 5 % bis 20 % der Schaftlänge. Bevorzugt wird der Schlauch 15 mit dem Schaft 14 in dem Mittelbereich 11 verklebt, insbesondere mittels Harzen wie bspw. Epoxid-, Polyester- oder Vinylesterharzen in einem Vakuumverfahren. Der Freibereich 16 des Schlauchs 15, welcher den Übergangsbereich 13 überlappt und über das erste Ende 17 des Schafts 14 übersteht, wird dabei nicht verklebt und klebstofffrei gehalten. Ein Verkleben kann an der Atmosphäre oder in einer evakuierten Vorrichtung erfolgen. Alternativ kann bei der Herstellung des Halbzeugs eine Aufnahmeschale 21 mit dem Schaft 14 verbunden werden, vorzugsweise verklebt.

Alternativ kann das Halbzeug 10 in einer entsprechenden Vorrichtung in einem Arbeitsschritt hergestellt werden. Dabei werden mehrere Schichten der Faserrovings, von denen wenigstens eine verlängerte oberste Schicht den späteren Schlauch 15 bildet, um einen Kern gelegt, welcher eine Innenkontur des Rohrs formt. Zudem wird eine Vorrichtung um diese Faserrovings positioniert, die den Mittelbereich 11 und den Übergangsbereich 13 des Schafts 14 umgibt und eine Aussparung für den Freibereich 16 des Schlauchs 15 aufweist. Anschließend kann das Harz in die Vorrichtung eingebracht werden, welche bevorzugt evakuiert wird. Der Freibereich 16 des Schlauchs 15 wird dabei nicht verklebt und harzfrei gehalten.

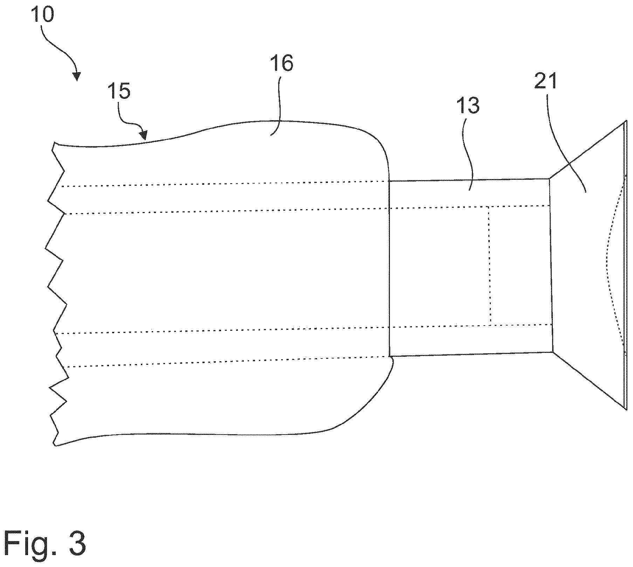

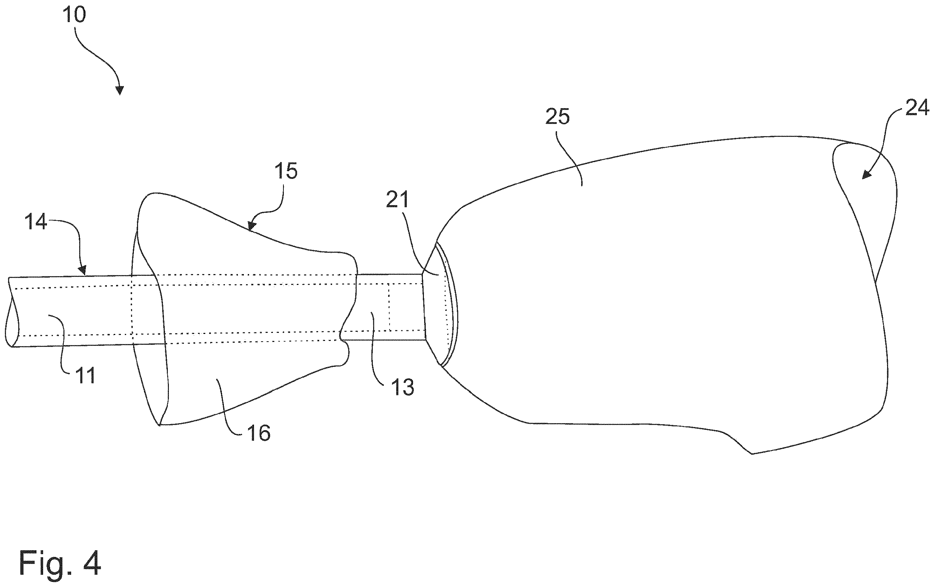

Zur Herstellung einer Unterschenkelprothese 1 gemäß Fig. 1 wird in einem ersten Arbeitsschritt der Freibereich 16 des Schlauchs 15 über den Mittelbereich 11 gestülpt, so dass der Übergangsbereich 16 und das erste Ende 17 des Schafts 14 freiliegen. Die Aufnahmeschale 21 wird mit dem ersten Ende 17 des Schafts 14 gemäß Fig. 3 verbunden. Bevorzugt wird ein zylindrischer Bereich der Aufnahmeschale 21 in eine korrespondierende Aussparung des Schafts 14 eingeführt und stoff- oder kraftschlüssig mit diesem verbunden, insbesondere verklebt. Alternativ kann die Aufnahmeschale 21 im Auslieferungszustand des Halbzeugs bereits mit dem Schaft 14 verbunden sein, so dass der Arbeitsschritt eines Verklebens der Aufnahmeschale 21 mit dem Schaft 14 entfällt. In einem weiteren Arbeitsschritt wird der Aufnahmeköcher 25 gemäß Fig. 4 in einer vorab ermittelten Position und Ausrichtung mit der Aufnahmeschale verbunden, bevorzugt wird er mit dieser verklebt. Hierfür können jeweils 2-K-Kleber verwendet werden.

Der Freibereich 16 des Schlauchs 15 wird in Richtung des ersten Endes 17 des Schafts 14 gezogen und über den Übergangsbereich 13, die Aufnahmeschale 21 und den Aufnahmeköcher 25 gelegt, bis er gemäß Fig. 5 an diesem anliegt. Der Schlauch 15 wird im Folgenden mit dem Übergangsbereich 13, der Aufnahmeschale 21 und dem Aufnahmeköcher 25 verbunden, bevorzugt in einer evakuierten Vorrichtung mittels eines Harzes verklebt. Dazu kann er auf an sich bekannte Art und Weise mit einem luftdichten Schlauch 15 überzogen werden, der dann eben evakuiert wird.

Am zweiten Ende 18 des Schafts wird gemäß Fig. 5 das Befestigungsteil 22 angebracht. Insbesondere kann dieses abschnittsweise mit dem zweiten Ende 18 des Schafts 14, also dem Mittelbereich 11 verklebt sein. In einem dem Mittelbereich 11 abgewandten Abschnitt des Befestigungsteils 22 wird die Fußprothese 23 mit dem Befestigungsteil 22 verbunden. Bevorzugt weist die Fußprothese ein Adapterelement auf, das mit den Justierelementen 26 des Befestigungsteils 22 zusammenwirkt und somit Möglichkeiten zur individuellen Einstellung und Anpassung an den Prothesenträger bietet.

## Patentansprüche

1. Halbzeug für eine Prothese, insbesondere für eine Unterschenkelprothese, mit einem längserstreckten biegesteifen Schaft, mit
- einem Schlauch aus Fasermaterial, welcher an dem Schaft angebracht ist und in wenigstens einem Mittelbereich des Schaftes mit dem Schaft unlösbar verbunden ist und
- einem freien, beweglichen und biegeschlaffen Freibereich des Schlauchs, welcher über mindestens ein erstes Ende des Schaftes übersteht.

2. Halbzeug nach Anspruch 1, **dadurch gekennzeichnet, dass** der Schaft an seinem ersten Ende einen Übergangsbereich aufweist, welcher sich über 5 % bis 20 % einer Schaftlänge erstreckt, und in welchem der Schlauch den Schaft frei, beweglich und biegeschlaff überlappt, wobei vorzugsweise der Übergangsbereich direkt an den Mittelbereich anschließt.

3. Halbzeug nach Anspruch 1 oder 2, **dadurch gekennzeichnet, dass** der Schlauch in dem Mittelbereich umlaufend eng an einer Innen- und/oder an einer Außenseite des Schafts anliegt bzw. an einer Außenseite anliegt und dabei den Schaft umgibt.

4. Halbzeug nach einem der vorhergehenden Ansprüche, **dadurch gekennzeichnet, dass** sich der Schlauch über den Mittelbereich des Schafts hinaus bis zu einem dem Freibereich abgewandten zweiten Ende des Schaftes erstreckt.

5. Halbzeug nach einem der Ansprüche 1 bis 3, **dadurch gekennzeichnet, dass** der Schlauch an dem zweiten Ende des Schafts ebenfalls mit einem freien, beweglichen und biegeschlaffen Freibereich über den Schaft übersteht, insbesondere mit einer überstehenden Länge von mindestens 10% der Länge des Schafts.

6. Halbzeug nach einem der vorhergehenden Ansprüche, **dadurch gekennzeichnet, dass** an dem Ende des Schafts mit dem Freibereich eine Aufnahmeschale mit einer Erstreckungsebene im Wesentlichen quer zur Längsrichtung des Schafts angeordnet ist, welche auf einer dem Schaft abgewandten Seite eine konkave Krümmung aufweist, wobei ein Durchmesser der Aufnahmeschale den Durchmesser des Schaftes übersteigt, vorzugsweise um 20 % bis 100 %.

7. Halbzeug nach Anspruch 6, **dadurch gekennzeichnet, dass** die Aufnahmeschale im Wesentlichen rotationssymmetrisch und dauerhaft mit dem Schaft verbunden ist, und dass eine konkave Vertiefung der Aufnahmeschale zwischen 10% und 30% des Durchmessers des Schafts beträgt.

8. Halbzeug nach Anspruch 5 und Anspruch 6 oder 7, **dadurch gekennzeichnet, dass** an dem zweiten Ende des Schafts ein Befestigungsteil angeordnet ist, das insbesondere am Schaft befestigt ist und einen größeren Querschnitt als der Schaft aufweist.

9. Halbzeug nach einem der vorhergehenden Ansprüche, **dadurch gekennzeichnet, dass** der Freibereich mit einer Länge von mindestens 20% der Länge des Schafts über das Ende des Schaftes übersteht, vorzugsweise mindestens 50%.

10. Prothese hergestellt mit einem Halbzeug nach einem der vorhergehenden Ansprüche, **dadurch gekennzeichnet, dass**
- am oberen Ende des Schafts ein Aufnahmeköcher für einen Gliedstumpf befestigt ist, und
- die Fasern des Freibereichs sich über mindestens 20% der Höhe des Aufnahmeköchers über eine Außenseite des Aufnahmeköchers erstrecken, wobei sie auf der Außenseite aufliegen und mit dieser verbunden sind.

11. Prothese nach Anspruch 10, **dadurch gekennzeichnet, dass** der Aufnahmeköcher mit einem ersten, unteren Ende an der Aufnahmeschale gemäß Anspruch 6 oder 7 anliegt, vorzugsweise mit dieser verklebt ist

12. Prothese nach Anspruch 10 oder 11, **dadurch gekennzeichnet, dass** an einem zweiten Ende des Schafts ein Fußteil befestigt ist, wobei die Fasern des unteren Freibereichs nach Anspruch 5 das Fußteil überlappen, vorzugsweise auf der Außenseite des Fußteils aufliegen und daran befestigt sind, wobei insbesondere das Fußteil in eine Fußhülle eingesteckt ist.

13. Verfahren zum Herstellen eines Halbzeugs nach einem der Ansprüche 1 bis 9, mit den Schritten:
- Bereitstellen des längserstreckten biegesteifen Schafts,
- Bereitstellen des Schlauchs aus Fasermaterial,
- Befestigen des Schlauchs auf dem Schaft in wenigstens dem Mittelbereich des Schafts, wobei der Schlauch mit dem freien, beweglichen und biegeschlaffen Freibereich über das erste Ende des Schafts übersteht.

14. Verfahren zum Herstellen eines Halbzeugs nach einem der Ansprüche 1 bis 9, mit den Schritten:
- Bereitstellen eines Schlauchs aus Fasermaterial,
- Herstellen eines längserstreckten biegesteifen Schafts aus einem Längsbereich des Schlauchs durch Verkleben,
- Freilassen des restlichen Längsbereichs des Schlauchs als freier, beweglicher und biegeschlaffer Freibereich, der über das erste Ende des Schafts übersteht.

15. Verfahren zur Herstellung einer Prothese nach einem der Ansprüche 10 bis 12, mit den Schritten:
- Befestigen des Aufnahmeköchers an dem freien Ende des Schafts in genau vorgegebener Position und Lage,
- Anlegen der Fasern des freien, beweglichen und biegeschlaffen Freibereichs an der Außenseite des Aufnahmeköchers,
- Verbinden des Freibereichs mit dem Schaft und dem Übergangsbereich sowie mit dem Aufnahmeköcher.
